# EUROPEAN PATENT APPLICATION

(11) **EP 4 840 877 A1**
(43) Date of publication of application: **18.11.2026**
(21) Application number: 24916443.5
(22) Date of filing: 18.09.2024
(51) Int. Cl.: C07K 14/435, C07K 14/47, C07K 16/18, C12N 15/11, C12N 15/62, A61K 38/10

(54) **PROTEIN KINASE A INHIBITOR PEPTIDE AND USE THEREOF**

(30) Priority: 09.01.2024 WO PCT/CN2024/071444
(71) Applicant: Chen, Xiongwen, Tianjin 300220 (CN)
(72) Inventor: Chen, Xiongwen, Tianjin 300220 (CN)
(74) Representative: Elzaburu S.L.P.
(86) International application number: PCT/CN2024/119331
(87) International publication number: WO 2025/148391

(57) **Abstract**

Involved are an engineered PKA inhibitor peptide, a dimer or a polymer formed by combining monomers. Also involved is a use of the PKA inhibitor peptide.

## Description

### TECHNICAL FIELD

The present application relates to the field of treatment of cardiovascular diseases, and in particular, to an inhibitor peptide that can inhibit protein kinase A, and a coding nucleic acid construct and a use thereof.

### BACKGROUND

Cardiomyocyte injury (caused by ischemia, hypertension, metabolic diseases, aortic valve regurgitation, or genetic diseases, for example) activates various neuroendocrine systems to maintain cardiac output and meet systemic blood perfusion demands by feedback. Activation of a sympathoadrenal system (SAS) is a core mechanism in the above process. Activation of the SAS system can increase a heart rate, enhance myocardial contractility, and accelerate cardiac relaxation, thereby achieving a goal of maintaining the cardiac output. However, sustained long-term activation of the SAS system induces adverse changes in myocardial morphology, structure, and function, namely, adverse remodeling, ultimately leading to heart failure and malignant arrhythmias. It is generally believed that catecholamines released by the SAS system act on cardiac β-receptors and activate downstream signaling pathways, including cAMP/PKA and Ca²⁺/CaM/CaMKII, which exert cardiotoxic effects and constitute one of the major causes of cardiac diseases.

Studies have shown that the modified protein kinase A inhibitor peptide (PKAi) of the patent corresponds to the protein kinase A (PKA) inhibitory domain derived from cAMP-dependent protein kinase inhibitor peptide α (PKIα). The modified PKAi can inhibit CaMKII activation by blocking cytoplasmic PKA activation and calcium overload upon the cardiomyocyte injury, and can also activate a protective cAMP/EPAC signaling pathway. Consequently, the modified PKAi protects cardiomyocytes and suppresses myocardial remodeling, thereby improving the structure and function of injured myocardium.

### SUMMARY

In the present disclosure, amino acids 1 to 25 of PKIα are selected as a base sequence for further engineering modification to enhance activity of the PKAi. To ensure stability of the PKAi, polypeptides that do not affect the activity of the PKAi are added to two termini of the PKAi, or two PKAi monomers are linked by a linker sequence to form a PKAi dimer, thereby achieving higher stability than conventional monomers. Moreover, to maximize efficacy of the PKAi while reducing its toxicity, a nuclear export sequence is added to the N terminus or the C terminus of a stable monomer or dimer of the PKAi. When specifically expressed in cardiomyocytes, a resulting construct can ameliorate cardiomyocyte injury, inhibit adverse remodeling, and promote functional recovery while reducing toxicity to the cardiomyocytes.

In one embodiment of the present disclosure, monomers in the PKAi dimer are linked therebetween by a covalent bond or a linker. The linker may be a cleavable linker or a non-cleavable linker. The cleavable linker may include, for example, RKRRKR (SEQ ID NO: 18), GSGATNFSLLKQAGDVEENPGP (SEQ ID NO: 6), and GFLG (SEQ ID NO: 19). The non-cleavable linker is also referred to as "internal linker", and includes, for example, GGGSGGGSGGGS (SEQ ID NO: 5), (GGGGS)₃ (SEQ ID NO: 12), (G)₈ (SEQ ID NO: 13), (G)₆ (SEQ ID NO: 14), (EAAAAK)₃ (SEQ ID NO: 15), PAPAP (SEQ ID NO: 16), AEAAAKEAAAKA (SEQ ID NO: 17), and a ((G)ₘ(S)ₙ)ₚ linker, where m, n, or p is selected from any positive integer in the range of 1 to 20. Preferably, the linker is a cleavable linker.

As used herein, the term "internal linker" refers to a chemical moiety having one end linked to one PKAi monomer and the other end linked to another PKAi monomer. In the present disclosure, the "internal linker" between the PKAi monomers may be an enzyme-stable linker.

In one embodiment of the present disclosure, the PKAi may form a multimer. In a first form of the multimer, a plurality of PKAi monomers are linked by the "internal linker". In a second form of the multimer, a plurality of PKAi monomers are linked by the "cleavable linker" (the "cleavable linker" is a polypeptide that covalently links the PKAi monomers and can be specifically and efficiently cleaved by an intracellular protease). In a third form of the multimer, a plurality of "PKAi dimers" are linked by the "cleavable linker". In a fourth form of the multimer, the PKAi monomers and the PKAi dimers are freely combined by the linker which may be the "internal linker" or the "cleavable linker".

In one embodiment of the present disclosure, the "cleavable linker" specifically refers to a linker that can be efficiently cleaved by an intracellular protease such as furin. This peptide linkage is considered highly stable during plasma circulation. This is because proteases are generally inactive extracellularly due to serum protease inhibitors. Given high plasma stability and favorable intracellular cleavage selectivity and efficiency, the "cleavable linker" includes a peptide with an intracellular enzyme-cleavable site. Specifically, the cleavable linker may be, for example, GSGATNFSLLKQAGDVEENPGP (SEQ ID NO: 6), GFLG (SEQ ID NO: 19), or KRRKR (SEQ ID NO: 20), which are cleavable by the intracellular protease such as the furin.

In one embodiment of the present disclosure, a nuclear export sequence (NES sequence) is added to the N terminus or the C terminus of the PKAi dimer to eliminate potential detrimental effects that may arise when the PKAi dimer is localized in a nucleus. The nuclear export sequence (also known as nuclear export signal, NES) is an amino acid sequence in a protein that includes four hydrophobic moieties and is responsible for the transport of a protein from the nucleus to cytoplasm through nuclear pores. The NES functions in opposition to a nuclear localization signal (NLS). The NLS is a domain present on the protein that is responsible for the import of a macromolecular protein into the nucleus. An NES fragment can be recognized and bound by an exportin. Upon binding of a to-be-transported protein, a Ran-exportin-target protein complex is formed and translocates across a nuclear envelope through the nuclear pores. In the present disclosure, the NES may be, for example, a sequence that marks a polypeptide for nuclear export, such as LQKKLEELEL (SEQ ID NO: 8), LECLNEQRLQGL (SEQ ID NO: 21), LRAARLRHQELFR (SEQ ID NO: 22), or LPPLERLTL (SEQ ID NO: 23).

### BRIEF DESCRIPTION OF THE DRAWINGS

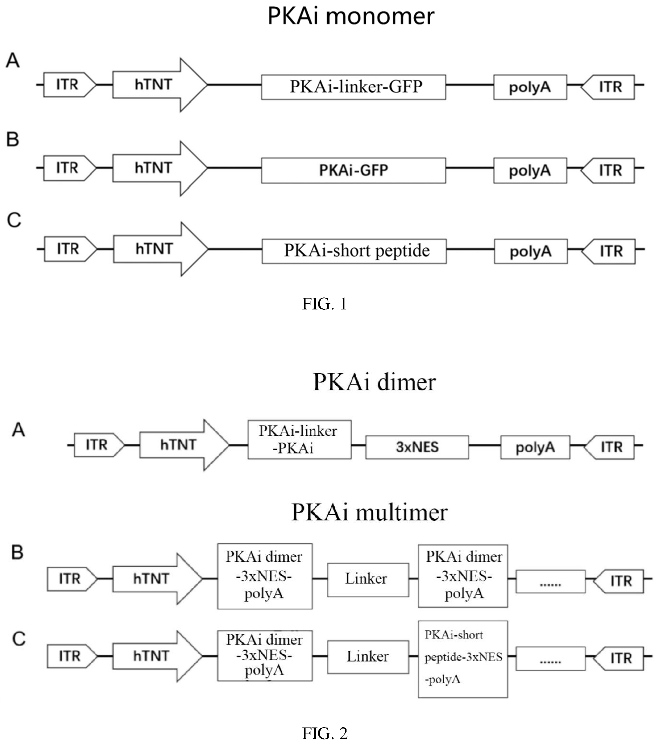

FIG. 1 shows artificially constructed cardiomyocyte-specific PKAi monomer expression vectors.
FIG. 2 shows artificially constructed cardiomyocyte-specific PKAi dimer or multimer expression vectors.

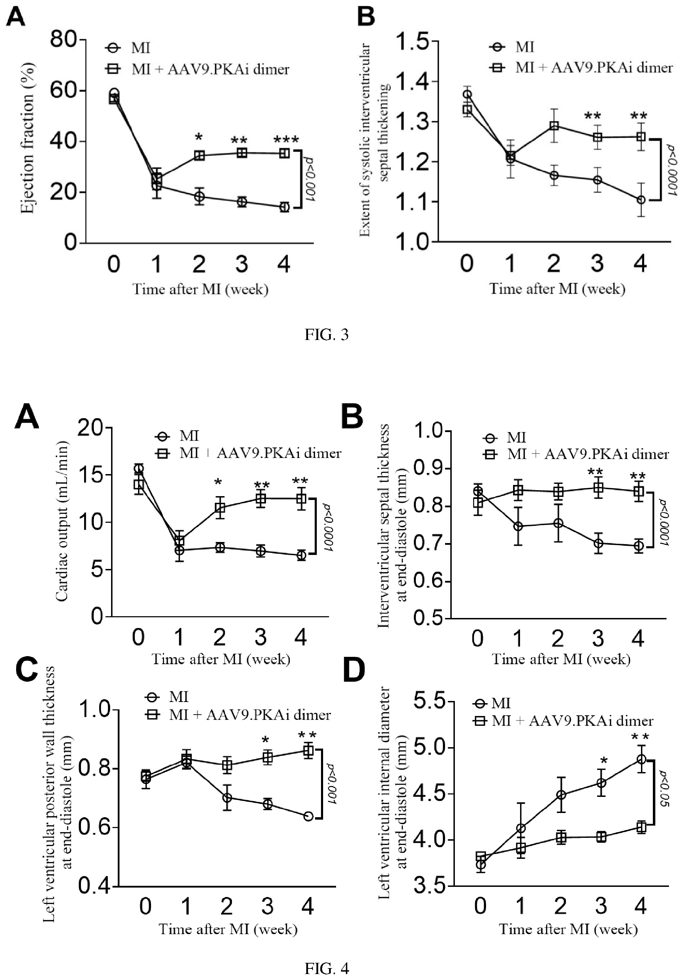

FIG. 3 shows preventive and therapeutic effects of AAV9.hTNT.PKAi-dimer-NES on heart failure following myocardial infarction (MI), where C57/b16 mice were each subjected to an MI surgery, and 1 d later, 5E10 vg per mouse was administered through tail vein injection; A shows ejection fractions of a treated group and a non-treated group. B shows left ventricular systolic wall thickening ratios of the treated group and the non-treated group; and *: *p* < 0.05 and **: *p* < 0.01, "MI + AAV9.PKAi-dimer" group vs. "MI" group.
FIG. 4 shows preventive and therapeutic effects of AAV9.hTNT.PKAi-dimer-NES on adverse cardiac remodeling following MI, where C57/b16 mice were each subjected to an MI surgery, and 1 d later, 5× 10¹⁰ vg per mouse was administered through tail vein injection; this figure illustrates changes in cardiac output (A), end-diastolic interventricular septal thicknesses (B), end-diastolic left ventricular posterior wall thicknesses (C), and end-diastolic left ventricular internal diameters (D) over time after the MI in a treated group and a non-treated group; and *: *p* < 0.05 and **: *p* < 0.01, "MI + AAV9.PKAi-dimer" group vs. "MI" group.

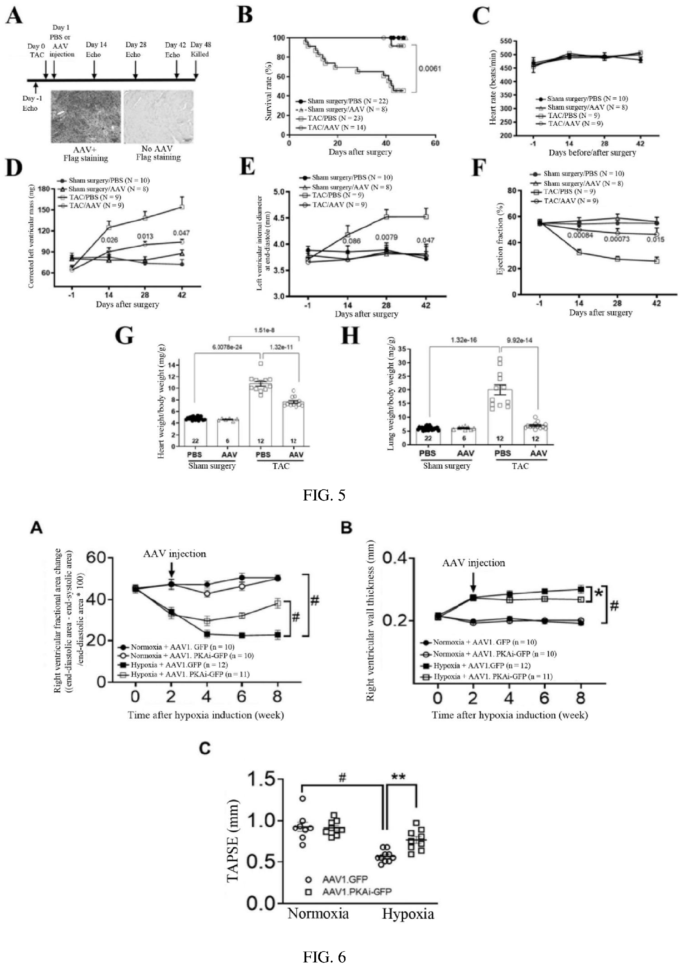

FIG. 5 shows preventive and therapeutic effects of AAV9.hTNT.PKAi-GFP on left ventricular hypertrophy and heart failure induced by pressure overload. A shows a mouse pressure overload model and a treatment regimen: mice were subjected to transaortic constriction (TAC, to increase cardiac pressure load) or sham surgery, and 1 d later, either phosphate buffered saline (PBS) or an adeno-associated virus (AAV) (2 × 10¹¹ vg per animal) was administered through tail vein injection; the lower panel in A shows FLAG staining after AAV infection (a 6 × FLAG sequence is inserted downstream of a green fluorescent protein (GFP)). B shows postoperative survival rates of the mice. C shows heart rates during echocardiography (ECHO). D shows corrected left ventricular masses measured by ECHO at different time points after the surgery; E shows end-diastolic left ventricular internal diameters at different time points after the surgery; F shows ejection fractions at different time points after the surgery. G shows heart weight-to-body weight ratios after sacrifice; H shows lung weight-to-body weight ratios after sacrifice; and numbers in this figure indicate p-values for a TAC/AAV-treated group compared with a TAC/PBS control group.
FIG. 6 shows preventive and therapeutic effects of AAV1.hTNT.PKAi-GFP on right ventricular hypertrophy and heart failure following hypoxia-induced pulmonary arterial hypertension (PAH). Mice were exposed to hypoxia for 2 h to induce PAH, and an AAV (5×10¹⁰ vg/animal) was then injected; a right ventricular structure and function are assessed weekly by ECHO. A shows right ventricular fractional area changes over time. B shows changes in right ventricular free wall thickness over time. C shows changes in tricuspid annular plane systolic excursion (TAPSE) over time; and * or # indicates a statistically significant difference between curves at *p* < 0.05 or *p* < 0.01.

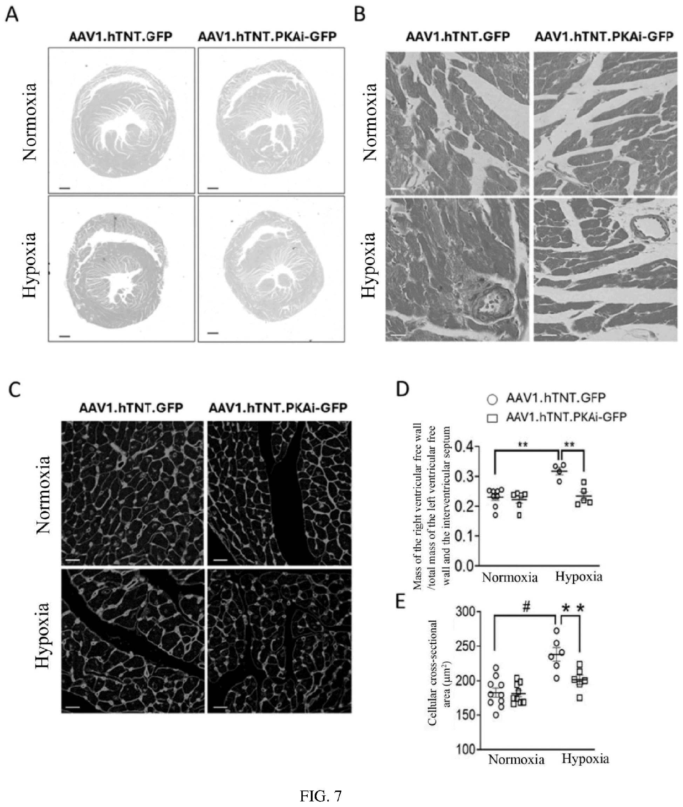

FIG. 7 shows preventive and therapeutic effects of AAV1.hTNT.PKAi-GFP on right ventricular fibrosis and hypertrophy following hypoxia-induced PAH, where after a PAH treatment, hearts were fixed, sectioned, and subjected to hematoxylin and eosin (H&E) staining, Masson's trichrome staining, or wheat germ agglutinin (WGA) staining. A shows H&E staining of mid-ventricular transverse sections; hypoxia can induce thickening of a right ventricular free wall (left two images), but this action is inhibited by AAV1-hTNT-PKAi-GFP (right two images); B shows magnified Masson's trichrome staining of the right ventricular free wall, indicating that hypoxia induces myocardial fibrosis in the AAV1-hTNT-GFP control-treated group, but AAV1-hTNT-PKAi-GFP prevents myocardial fibrosis. C shows WGA staining of cardiomyocytes: hypoxia induces an increase in cardiomyocyte cross-sectional area in the AAV1-hTNT-GFP control-treated group, but AAV1-hTNT-PKAi-GFP prevents this change. D shows a ratio of a right ventricular free wall mass to a total left ventricular mass (left ventricular free wall mass + interventricular septal mass): the hypoxia increases this ratio in the control-treated group, indicating right ventricular hypertrophy, which is attenuated by AAV1.PKAi-GFP; and E shows that the cardiomyocyte cross-sectional area increases in response to the hypoxia in the control-treated group (AAV1.GFP), but AAV1.PKAi-GFP prevents such an increase.

### DETAILED DESCRIPTION OF THE EMBODIMENTS

In view of the deficiencies of the prior art, an objective of the present disclosure is to provide an expression cassette for stably and efficiently expressing PKAi capable of treating a cardiac disease.

The second objective of the present disclosure is to provide a vector and a vector system including the expression cassette.

The third objective of the present disclosure is to provide a recombinant adeno-associated virus (AAV) expressing the protein kinase inhibitor peptide and a composition thereof.

The fourth objective of the present disclosure is to provide a use of the expression cassette, the vector, or the recombinant AAV in treating a cardiac disease.

The objectives of the present disclosure can be achieved by the following technical solutions:
A. The protein kinase A inhibitor peptide (abbreviated as PKAi in the full text) of the present disclosure includes a PKA inhibitory domain of PKIα, such as the amino acid sequence of amino acids 1 to 25 as shown in SEQ ID NO: 1, which is derived from a human PKAi protein sequence (SEQ ID NO: 11) and has been further modified.

In some embodiments, based on the modified PKAi of the present disclosure, as a first PKAi, a linker (for example, SEQ ID NO: 5 or SEQ ID NO: 6) may be further added, and a second PKAi may be linked downstream of the linker to produce a dimer (for example, SEQ ID NO: 7). The PKAi sequences involved may be any two variants based on SEQ ID NO: 1. A nucleotide sequence encoding the second PKAi does not include a start codon but includes a stop codon. In some embodiments, based on the modified PKAi of the present disclosure, as the first PKAi, the linker (for example, SEQ ID NO: 5 or SEQ ID NO: 6) may be further added, and a second PKAi, a third PKAi, a fourth PKAi, a fifth PKAi, a sixth PKAi, a seventh PKAi, an eighth PKAi, etc. may be linked downstream of the linker to produce a dimer (for example, SEQ ID NO: 7), a trimer, a tetramer, a pentamer, a hexamer, a heptamer, an octamer, or a higher-order multimer. The PKAi sequences involved may be any variants based on SEQ ID NO: 1 (sequence 1).

As a preferred embodiment of the present disclosure, tyrosine at position 8 of the PKAi sequence is mutated to phenylalanine (SEQ ID NO: 2). Studies have demonstrated that this mutation enhances activity and stability of the PKAi.

As a preferred embodiment of the present disclosure, serine at position 14 of the PKAi sequence is mutated to cysteine (SEQ ID NO: 3). Studies have demonstrated that this mutation enhances the activity and the stability of the PKAi.

As a preferred embodiment of the present disclosure, in the PKAi sequence, the tyrosine at position 8 is mutated to the phenylalanine, and the serine at position 14 is mutated to the cysteine (SEQ ID NO: 4), which further enhances the activity and the stability of the PKAi.

B. To enable better cardiac expression of the PKAi, a human troponin T (TNT) or brain natriuretic peptide (BNP) promoter is used in construction of the expression cassette. Studies have proved that the TNT or BNP promoter can specifically initiate expression of the PKAi in cardiomyocytes or at specific disease stages.

C. As a preferred embodiment of the present disclosure, three tandem NESs (as shown in SEQ ID NO: 8) are added downstream of the PKAi dimer sequence. Studies have found that this solution enhances the function of the PKAi and mitigates potential toxicity of the PKAi.

D. A WPRE sequence is added downstream of the PKAi dimer to maintain stability of the PKAi dimer.

E. The vector used for the expression cassette includes, but is not limited to, AAV1, AAV2, AAV3, AAV4, AAV5, AAV6, AAV7, AAV8, AAV9, AAV10, AAV11, AAV12, or any other AAV vectors known in the art or discovered in the future.

In the patent, a cardiac-specific promoter is employed to enhance specificity, efficiency, and disease-stage responsiveness of gene expression in the cardiomyocytes. In addition, the gene used in the patent is an upstream regulatory molecule capable of modulating a plurality of cardiac function-regulating genes by inhibiting PKA, including SERCA2a, L-type calcium channels, CaMKII, etc. Therefore, a very low viral dose is sufficient to achieve a potent regulatory effect.

Currently, synthetic PKAi analogues include: PKAi-(6-22)-amide (Thr-Tyr-Ala-Asp-Phe-Ile-Ala-Ser-Gly-Arg-Thr-Gly-Arg-Arg-Asn-Ala-Ile-NH2) (Anal.Bioanal.Chem.381, 647-655.doi: 10.1007/s00216-005-3070-2), PKAi-(14-24)-amide (Gly-Arg-Thr-Gly-Arg-Arg-Asn-Ala-Ile-His-Asp-NH2) (Apoptosis 11, 1263-1273.doi: 10.1007/s10495-006-7702-6), and PKAi-(5-24)-amide (Thr-Thr-Tyr-Ala-Asp-Phelle-Ala-Ser-Gly-Arg-Thr-Gly-Arg-Arg-Asn-Ala-Ile-His-Asp-NH2) (Gen.Comp.Endocrinol.181, 88-97.doi: 10.1016/j.ygcen.2012.10.016). These synthetic PKAi analogues all suffer from poor intracellular stability and short duration of action, and exhibit PKA inhibitory efficiencies ranking from highest to lowest as follows: PKAi-(6-22) > PKAi-(5-24) > PKAi-(14-24).

Existing commercially available PKAi sequences include PKAi-(6-22), PKAi-(5-24), and PKAi-(14-24) in three distinct lengths, all of which are synthetically produced.

The PKIα includes 75 amino acids, of which amino acids 1 to 25 constitute a PKA inhibitory domain (PKAi) of the PKIα.

Currently, recombinant AAVs are prepared and purified as follows: Helper elements, a capsid protein, and genomic DNA including an expression cassette of a target gene are co-expressed in a human embryonic kidney cell line (HEK293). Viral particles are continuously packaged in the human embryonic kidney cell line. Cell lysis and viral extraction are then conducted. In a laboratory, purification is conducted through iodixanol density gradient ultracentrifugation to produce usable viral particles. During large-scale production, a column chromatography-based separation and purification method is employed.

### Examples

### 1. Materials and methods

### 1.1 Instrument information

| **Instruments and equipment** | **Country/manufacturer** | **Model** |
|---|---|---|
| Anesthesia machine | Shanghai Renyi Biotechnology Co., Ltd. | MSS-3 |
| Stereomicroscope | American AM SCOPE | SM-4TZ-144-10M |
| Ventilator | Harvard Apparatus, USA | MiniVent TYPE845 |
| Ultra-high-frequency small-animal ultrasound system | FUJIFILM VisualSonics, Canada | Vevo2100 |
| Ultracentrifuge | Beckman Coulter, USA | Optimal-100XP |
| Biosafety cabinet | Suzhou Redian | 1389 |
| Clean bench | Shanghai ZHICHENG | ZHJH-C1112B |
| CO₂ incubator | Thermo Fisher Scientific, USA | 150i |
| Biochemical incubator | ZHONGYIGUOKE, Beijing | SPX-70 |
| Incubator shaker | Shanghai ZHICHENG | ZWY-240 |
| Vacuum pump | Haimen Kylin-Bell Lab Instruments Co., Ltd., Jiangsu | GL-802B |
| Inverted fluorescence microscope | Olympus Corporation, Japan | IX73 |
| Nucleic acid/protein analyzer | Thermo Fisher Scientific, USA | nano ONE |
| PCR instrument | Bio-Rad Laboratories, USA | S1000 |
| Pipette | Eppendorf, Germany | Research Plus |
| Electric pipette controller | Eppendorf, Germany | Easypet 3 |
| Electronic pipette | Eppendorf, Germany | Multipette E3 |
| Refrigerated high-speed centrifuge | Thermo Fisher Scientific, USA | ST16R |
| Dry bath | Hangzhou Allsheng Instruments Co., Ltd. | K30 |
| Water bath (three-well) | Shanghai Yiheng Technology Instrument Co., Ltd. | DK-8D |
| Vortexer | Haimen Kylin-Bell Lab Instruments Co., Ltd., Jiangsu | VOTEX5 |
| Analytical balance | Mettler-Toledo Instruments (Shanghai) Co., Ltd. | ME204/02 |
| Heating magnetic stirrer | Haimen Kylin-Bell Lab Instruments Co., Ltd., Jiangsu | GL-3250A |
| pH meter | Mettler-Toledo Instruments (Shanghai) Co., Ltd. | FE28-Standard |

### 1.2 Reagent information

| **Name** | **Brand** | **Cat No.** |
|---|---|---|
| Endofree Plasmid Maxi Kit | TIANGEN BIOTECH (BEIJING) CO., LTD. | DP117 |
| Competent cell Stb13 | Beijing Zoman Biotechnology Co. Ltd. | ZC108-2 |
| LB Broth Base | Invitrogen | 12795084 |
| LB Agar | Invitrogen | 30391023 |
| Polyethylenimine (PEI) | Yeasen | 40816ESO3 |
| 60% iodixanol stock solution | STEMCELL | 07820 |
| Fetal bovine serum | BI | 04-001-1A |
| Penicillin/streptomycin | Gibco | 15070063 |
| DMEM Basic Medium | Gibco | C11965500bt |
| Salt active nuclease (SAN) | Sigma | SRE0015 |
| Polyethylene glycol (PEG) 8000 | Sigma | 89510-1KG-F |
| Pluronic F-68 (10%) | Gibco | 24040-032 |
| Deoxyribonuclease I | Solarbio | D8071 |
| Proteinase K | Solarbio | P9460 |
| Sodium N-lauroylsarcosinate | Solarbio | S8100 |
| Sodium chloride | Solarbio | S8210 |
| Magnesium chloride hexahydrate | Sigma | M2393-100G |
| Ethylenediaminetetraacetic acid (EDTA) | Sigma | E5134-100G |
| Potassium chloride | Sigma | P9541-500G |
| 0.25% Trypsin | Gibco | 25200056 |
| Calcium chloride dihydrate | Sigma | C3306-500G |
| Ampicillin | Solarbio | A8180 |
| PBS | Solarbio | P1010 |
| Phenol red | Solarbio | P8460 |
| SYBR Green Master Mix | Yeasen | 11184ESO3 |
| Sterile, Nuclease-Free Water | Solarbio | R1600 |

### 1.3 Detailed experimental methods

### 1.3.1 Plasmid construction

An optimized AAV backbone plasmid, a vector plasmid pAAV.hTNT.PKAi-GFP.SV40, pAAV.hTNT.GFP.SV40, or pAAV.hTNT.PKAi dimer. SV40, pAAV2/1 or pAAV2/9n, and a helper plasmid pAdDeltaF6 were all artificially synthesized as full-length fragments.

### 1.3.2 Viral packaging and purification

An AAV was prepared according to the experimental method described by Grieger et al. (Nat Protoc. 2006; 1(3): 1412-28).

### 1.3.3 AAV genome extraction and titer determination

100 µL of a DNase lysis solution (50 U DNase I + 1 mL DNase lysis buffer) was added to 2 µL of a viral solution.

A resulting mixed solution was mixed for 2 s, avoiding vigorous mixing to prevent damage to viral capsid. Incubation was conducted in a water bath at 37°C for 1 h.

5 µL of EDTA was added, and a resulting mixture was thoroughly mixed and incubated in a dry bath at 70°C for 10 min.

120 µL of a proteinase lysis solution (1.818 U proteinase K + 500 µL proteinase K solution) was added, and a resulting system was thoroughly mixed, centrifuged briefly for 3 s, and incubated in a dry bath at 55°C for 2 h.

The dry bath was set to 95°C, and incubation was continued for 10 min to inactivate the proteinase K. Cooling was then conducted on ice.

3 µL of a resulting sample was diluted 300-fold and then tested for a titer.

The extracted DNA was quantified by RT-PCR using a standard plasmid to determine an AAV titer.

### 1.3.4 ECHO

MI model: On day 2 after an MI surgery, AAV9.hTNT.PKAi-dimer-NES was injected at a dose of 5 × 10¹⁰ vg/mouse. ECHO was performed weekly after the surgery to evaluate a left ventricular function.

PAH model: Two weeks after the hypoxia-induced PAH model was established, AAV1.hTNT.PKAi-GFP was injected at a dose of 5E10 vg/mouse. ECHO was performed every two weeks starting from model establishment.

### Example 1: Optimization of a gene sequence for human PKAi and vector construction

A TNT promoter sequence was obtained from NCBI Reference Sequence: NG_007556.1 (4499bp-5042bp, as shown in SEQ ID NO: 9). A coding sequence for amino acids 1 to 25 of human PKAi was optimized and then synthesized as a full-length gene to produce a pAAV-hTNT-PKAi-dimer-3NES vector (as shown in FIG. 2). Moreover, a PKAi multimer (such as a tetramer or a hexamer) could be prepared with PKAi-dimer-NES as a unit, where units were linked by a short peptide sequence cleavable by an intracellular protease. Alternatively, a multimer could be formed by combination of PKAi-dimer-NES units and PKAi-monomer units (as shown in FIG. 2).

### Example 2: Packaging and purification of AAV9.hTNT-PKAi-dimer-NES virus

Viral packaging, purification, and titer determination methods reported by Grieger et al. (Nat Protoc. 2006; 1(3): 1412-28) and Rosemary C. Challis (dx.doi.org/10.17504/protocols.io.84ahyse) were adopted. A Rep-Cap expression plasmid (pAAV2/9), a helper plasmid (pAd△F6), and a PKAi expression plasmid (pAAV-hTNT-PKAi-dimer-NES) were co-transfected into HEK293T cells using PEI for expression and packaging of viral components. Transfected cells were cultured for 96 h. A resulting cell culture supernatant and cells were collected separately. The cell culture supernatant was subjected to precipitation with PEG 8000 to produce a viral pellet. The cells were subjected to lysis to produce a cell lysate. The viral pellet and the cell lysate were mixed and centrifuged to produce a crude viral solution. Virus purification was carried out by iodixanol density gradient centrifugation. A titer was determined by qPCR.

### Example 3: Establishment and treatment of MI model

A mouse MI model was established. After a surgery, an AAV9.PKAi dimer (SEQ ID NO: 7)-NES virus (5E10 vg/mouse) was injected. ECHO was then performed weekly. At week 4, it was observed that an ejection fraction, a systolic-to-diastolic interventricular septal thickness ratio (namely, an extent of systolic interventricular septal thickening), and a cardiac output were significantly improved in mice of a treated group (as shown in FIG. 3A, FIG. 3B, and FIG. 4A). During left ventricular structural assessment, ECHO showed that thicknesses of interventricular septum and left ventricular posterior wall at end-diastole did not significantly change in the treated group, but were reduced in a non-treated group (as shown in FIG. 4B and FIG. 4C). MI led to an increase in left ventricular internal diameter. It was found that left ventricular dilatation was ameliorated in the treated group (as shown in FIG. 4D). The above results indicated that the PKAi dimer could ameliorate cardiac dysfunction and remodeling induced by the MI.

### Example 4: Construction of a human PKAi-GFP fusion expression vector

A hTNT promoter sequence was obtained from NCBI Reference Sequence: NG_007556.1 (4499 bp - 5042bp). A coding sequence for amino acids 1 to 25 of human PKAi was synthesized and linked to a full-length GFP sequence by a linker. A pAAV-hTNT-PKAi-GFP vector was constructed through restriction enzyme digestion and ligation (as shown in FIG. 1A). A control GFP expression vector pAAV-hTNT-GFP was also constructed.

### Example 5: Packaging and purification of AAV1-hTNT-PKAi-GFP or AAV9-hTNT-PKAi-GFP virus

A Rep-Cap expression plasmid (pAAV2/1 or pAAV2/9n), a helper plasmid (pAd△F6), and a PKAi expression plasmid (pAAV-hTNT-PKAi-GFP) were co-transfected into HEK293T cells using PEI for expression and packaging of viral components. Transfected cells were cultured for 96 h. A resulting cell culture supernatant and cells were collected separately. The cell culture supernatant was subjected to precipitation with PEG 8000 to produce a viral pellet. The cells were subjected to lysis to produce a cell lysate. The viral pellet and the cell lysate were mixed and centrifuged to produce a crude viral solution. Virus purification was carried out by iodixanol density gradient centrifugation. A titer was determined by qPCR.

### Example 6: Establishment and treatment of TAC-induced left ventricular hypertrophy and heart failure model

An ascending aortic constriction model was established by a surgery to increase left ventricular afterload, thereby inducing left ventricular hypertrophy and heart failure. Subsequently, gene therapy was applied with AAV9.hTNT.PKAi (SEQ ID NO: 1)-GFP to reverse cardiac hypertrophy and prevent heart failure. A median sternotomy was performed to expose a transverse aortic arch. A 26-gauge needle was placed alongside an aorta between a right brachiocephalic artery and a left common carotid artery, and ligation was then conducted using 7-0 suture. The 26-gauge needle was then withdrawn to create aortic constriction and elevate left ventricular afterload. A sham surgery was performed on animals as a control for TAC. After the surgery, AAV9.hTNT.PKAi-GFP was injected through a tail vein at a dose of 2 × 10¹¹ vg/mouse. Subsequently, the animals were monitored for 6 weeks. Animal survival was checked daily. ECHO was performed weekly on anesthetized mice (1.5% isoflurane) using a VisualSonicsVevo 2100 system (FUJIFILM visualsonic, Toronto, Canada) to assess a cardiac function and morphology. A baseline heart rate during ECHO was maintained between 400 and 450 beats per minute (FIG. 5C). Immunohistochemical staining showed that 95% or more of cardiomyocytes could be infected to express PKAi-GFP (FIG. 5A). The survival rate of mice treated with AAV9.hTNT.PKAi-GFP was significantly higher than that of untreated mice (FIG. 5B). With heart rates comparable between the groups (FIG. 5C), the gene therapy significantly reduced or prevented a TAC-induced increase in left ventricular mass (FIG. 5D), a TAC-induced increase in left ventricular internal diameter at end-diastole (FIG. 5E), a TAC-induced reduction in ejection fraction, namely, heart failure (FIG. 5F), a TAC-induced increase in heart weight-to-body weight ratio (FIG. 5G), and a TAC-induced increase in lung weight-to-body weight ratio (FIG. 5H). Numbers in this figure indicate p-values for the TAC/AAV-treated group compared with the TAC/PBS control group.

### Example 7: Establishment and treatment of SU5416/hypoxia combination-induced PAH model

An SU5416/hypoxia combination-induced PAH model was established, and then subjected to gene therapy with either AAV1-hTNT-PKAi (SEQ ID NO: 2)-GFP or AAV1.hTNT-GFP (AAV dose: 5E10 vg/mouse). ECHO was performed at weeks 0, 2, 4, 6, and 8. It was observed that AAV1.hTNT-PKAi-GFP significantly improved PAH-induced right ventricular dysfunction (namely, a reduction in right ventricular fractional area change) (FIG. 6A). In addition, PAH-induced thickening of the right ventricular free wall was also significantly ameliorated (FIG. 6B). ECHO analysis at week 8 revealed that right ventricular tricuspid annular plane systolic excursion also significantly increased after PKAi-GFP therapy (FIG. 6C).

After 8 weeks, samples were collected from mice, and hearts were weighed. Right ventricles, left ventricles, and interventricular septums were weighed separately. It was found that a ratio of the weight of the right ventricle to the total weight of the left ventricle and the interventricular septum of mice significantly increased in the PAH group, but significantly decreased after PKAi-GFP therapy (FIG. 7D). Consistent results were also observed in H&E-stained fixed cardiac sections. A right ventricular wall thickness increased after PAH, which was ameliorated after PKAi-GFP gene therapy (FIG. 7A).

Cardiac tissue sections were prepared and subjected to Masson's trichrome staining. It was found that no obvious fibrosis was observed in either viral injection group under normoxic conditions. The PAH led to increased fibrosis of the right ventricle. However, after PKAi gene therapy, a fibrotic area of the right ventricle was reduced (FIG. 7B).

WGA could specifically bind to glycoproteins on the cardiomyocyte membrane, thereby staining the cardiomyocyte membrane. Cardiomyocyte sections were subjected to WGA staining (FIG. 7C). An area of DAPI-stained cells was quantified, with at least 4 fields of view and a total of 100 cells counted per tissue. An average value was calculated. According to statistical results, right ventricular cardiomyocytes underwent significant hypertrophy after the PAH, whereas the hypertrophy was markedly ameliorated after AAV1.hTNT.PKAi-GFP therapy (FIG. 7E).

### Sequence Listing

| SEQ ID NO. | Sequence description | Specific sequence |
|---|---|---|
| 1 | Amino acid sequence of PKAi monomer | MTDVETTYADFIASGRTGRRNAIHD |
| 2 | Amino acid sequence of PKAi mutant monomer | MTDVETTFADFIASGRTGRRNAIHD |
| 3 | Amino acid sequence of PKAi mutant monomer | MTDVETTYADFIACGRTGRRNAIHD |
| 4 | Amino acid sequence of PKAi mutant monomer | MTDVETTFADFIACGRTGRRNAIHD |
| 5 | Amino acid sequence of linker in dimer | GGGSGGGSGGGS |
| 6 | Linker cleavable by protease | GSGATNFSLLKQAGDVEENPGP |
| 7 | Amino acid sequence of PKAi dimer | MTDVETTYADFIASGRTGRRNAIHD-TDVETTY ADFIASGRTGRRNAIHD wherein "-" represents GGGSGGGSGGG |
| 8 | NES sequence | LQKKLEELEL |
| 9 | hTNT promoter | ctcagtccattaggagccagtagcctggaagatgtctttacccccagcatc agttcaagtggagcagcacataactcttgccctctgccttccaagattctgg tgctgagacttatggagtgtcttggaggttgccttctgcccccaaccctgc tcccagctggccctcccaggcctgggttgctggcctctgctttatcaggatt ctcaagagggacagctggtttatgttgcatgactgttccctgcatatctgct ctggttttaaatagcttatctgagcagctggaggaccacatgggcttatatg gcgtggggtacatgttcctgtagccttgtccctggcacctgccaaaatagc agccaacacccccccacccccaccgccatccccctgccccacccgtccc ctgtcgcacattcctccctccgcagggctggctcaccaggccccagccc acatgcctgcttaaagccctctccatcctctgcctcacccagtccccgctg agactgagcagacgcctccaggatctgtcggcag |
| 10 | hBNP promoter | atcagatctctctgtcaaccaggctggagtgcagtggcgtgatctcagc tcactgcaacctccacctcctgggttcaagtgattctccttcctcagcctcc cgagtagctgggactacaggcgcctgccaccatgcccagctaatttttgg taattttagtagagacggggtttcaccgtggtctcgatctcctgacctcgtg atccgaccgcctcggcctctcaaagtgctgggattacaggcgtgaacca ccatgcccagcctatccttttgttttccatcctgtgttggcttggtgggggag aggaggtgttgacacgtggaggacacacatataaggcattcttgggtgac ttcgtcatcactggaccctatctctcaaaattccagcgaaatctgctcttccc tttaaggagtgaaagaagggtcagcattccagaagttcctggtcataccca |
| | | ggcttttaatgaattgccactggggaatcagcatcccgttgctgtaaggact ataagatggcggattgtgagagcatagggaaaggtctcggaggtctcttg tccttgctccacgcaggtctttctggcctgaaaatcccgttgaagagagca gctcttgagagtttgctccaagttccctcggggtgatcagcaccacggac agcggccagggcgcccccgaggacccgcaggcaggcagggtgcaca gcggcgagcaggtgctgcgctacgtgcgggccagggaactcgcgcgg ggaggggagaggcgccgcgggtggcggggtcttggccggggctgtttt cgctgtgagtcaccccgtgctccccgcgctcacgtcggtcctcggaaag ccggggtcctccctgccttttccagcaacggtggggtggggaggcagga agaaagcgccaacctaggacacctggacatttgcaggaaaggaagaag cgggagacggggacttgtctgtgtctccagcgcgttcctgcccccggc ccgacccggcccatttctatacaaggtcggctctgcccggtctccacctcc cacgtcgcaggcgcggaggggctcattcccgggccctgatctcagagg cccggaatgtggctgataaatcagagataaccctgcatggcagggcagg cccgacactcagctccaggataaaggccacggtgtcccgaggagcca ggaggagcaccccgcaggctgagggcaggtgggaagcaaacccgga cgcatcgcagcagcagcagcagcagcagaagcagcagcagcagcctc cgcagtccctccagagac |
| 11 | Full-length amino acid sequence of PKIα | Human: |
| | | MTDVETTYADFIASGRTGRRNAIHDILVSSASG NSNELALKLAGLDINKTEGEEDAQRSSTEQSGE AQGEAAKSES PKIα sequences in other animals are highly conserved |
| 12 | Amino acid sequence of internal linker | (GGGGS)₃ |
| 13 | Amino acid sequence of internal linker | (G)₈ |
| 14 | Amino acid sequence of internal linker | (G)₆ |
| 15 | Amino acid sequence of internal linker | (EAAAAK)₃ |
| 16 | Amino acid sequence of internal linker | PAPAP |
| 17 | Amino acid sequence of internal linker | AEAAAKEAAAKA |
| 18 | Amino acid sequence of internal linker | RKRRKR |
| 19 | Linker cleavable by protease | GFLG |
| 20 | Linker cleavable by protease | KRRKR |
| 21 | NES sequence | LECLNEQRLQGL |
| 22 | NES sequence | LRAARLRHQELFR |
| 23 | NES sequence | LPPLERLTL |

| | | |
|---|---|---|
| Notes: Each polynucleotide sequence in the above sequence listing may represent either a DNA sequence or an RNA sequence. In the case of the polynucleotide sequence representing the RNA sequence, t in the polynucleotide sequence corresponds to uracil. | | |

## Claims

1. A protein kinase A inhibitor peptide (PKAi) monomer, comprising a protein kinase A inhibitory domain as shown in the formula (I):
X₀TDVETTX₁ADX₂IAX₃GRTGRRNAIHD (I),
wherein, X₀ is absent or is methionine (M), and X₁, X₂, and X₃ are each independently any amino acid.

2. The protein kinase A inhibitor peptide (PKAi) monomer according to claim 2, wherein:
X₁ is threonine (T), serine (S), tyrosine (Y), tryptophan (W), phenylalanine (F), or alanine (A);
X₂ is tyrosine (Y), phenylalanine (F), alanine (A), valine (V), leucine (L), or isoleucine (I); and
X₃ is serine (S), threonine (T), or cysteine (C); and preferably the engineered PKAi monomer comprises one of SEQ ID NOs: 1-4.

3. The protein kinase A inhibitor peptide (PKAi) monomer according to claim 1 or 2, further comprising at least one, two, or three nuclear export sequences (NES) at the N terminus and/or the C terminus, and preferably, the NES comprises the amino acid sequence as shown in SEQ ID NO: 8.

4. A multimer formed from the protein kinase A inhibitor peptide (PKAi) monomer according to any one of claims 1 to 3, wherein the multimer is a dimer, a trimer, a tetramer, a pentamer, a hexamer, or a higher-order multimer of the monomer.

5. The multimer according to claim 4, wherein protein kinase A inhibitor peptide (PKAi) monomers are linked therebetween by a covalent bond or a linker.

6. The multimer according to claim 5, wherein the linker is selected from 2A peptide and ((G)ₘ(S)ₙ)ₚ, wherein m, n, or p is independently selected from any positive integer in the range of 1 to 20.

7. The multimer according to claim 5, wherein the linker comprises a peptide with an intracellular enzyme-cleavable site.

8. The multimer according to claim 7, wherein the peptide with the intracellular enzyme-cleavable site comprises any one or more of SEQ ID NOs: 6 and 19-20.

9. The multimer according to any one of claims 3 to 7, comprising the amino acid sequence as shown in any one of SEQ ID NOs: 1-4.

10. The multimer according to any one of claims 4 to 10, further comprising at least one, two, three, or more nuclear export sequences (NES) at the N terminus or the C terminus, and preferably, the NES comprises the amino acid sequence as shown in SEQ ID NO: 8.

11. A nucleic acid molecule, comprising a coding nucleic acid sequence of the protein kinase A inhibitor peptide (PKAi) monomer according to any one of claims 1 to 3 or the multimer according to any one of claims 4 to 10.

12. The nucleic acid molecule according to claim 11, further comprising a nucleic acid sequence of a myocardial-specific or non-specific promoter at the 5' terminus of the coding nucleic acid sequence, and preferably, the promoter is selected from one or more of the following myocardial-specific promoters: a troponin T (TNT) promoter, a brain natriuretic peptide (BNP) promoter, a myosin light chain 2v (MLC2v) promoter, and an α-myosin heavy chain promoter.

13. The nucleic acid molecule according to claim 12, wherein the promoter comprises the polynucleotide sequence as shown in SEQ ID NO: 9 or SEQ ID NO: 10.

14. The nucleic acid molecule according to any one of claims 11 to 13, further comprising a coding sequence of WPRE at the 3' terminus of the coding nucleic acid sequence.

15. A genetically engineered vector, comprising the nucleic acid molecule according to any one of claims 11 to 14 or a complementary sequence thereof, and preferably, the vector is a plasmid or a viral vector.

16. The genetically engineered vector according to claim 15, wherein the viral vector is a lentiviral vector or an adeno-associated virus (AAV) vector, such as AAV1, AAV8, or AAV9.

17. A genetically engineered cell, comprising the nucleic acid molecule according to any one of claims 11 to 14 or the genetically engineered vector according to claim 15 or 16.

18. A pharmaceutical composition, comprising the protein kinase A inhibitor peptide (PKAi) monomer according to any one of claims 1 to 3 or the multimer according to any one of claims 4 to 10, and a pharmaceutically acceptable carrier or excipient.

19. A method for preventing or treating a myocardial injury disease, comprising administering an effective amount of the pharmaceutical composition according to claim 18 to a subject, and preferably, the myocardial injury disease is a myocardial injury disease caused by a causative factor selected from ischemia, arterial hypertension, metabolic disorder, bacterial or viral infection, and genetic factor.

20. The method according to claim 19, wherein the myocardial injury disease is selected from one or more of heart failure, myocardial infarction, cardiac hypertrophy, hypertension, pulmonary arterial hypertension, sepsis, and diabetes.
